# EUROPEAN PATENT APPLICATION

(11) **EP 1 345 150 A2**
(43) Date of publication of application: **17.09.2003**
(21) Application number: 02025419.9
(22) Date of filing: 14.11.2002
(51) Int. Cl.: G06F 19/00

(54) **Statistical-medical-information supplying method and apparatus**

(30) Priority: 12.03.2002 JP 2002066565
(71) Applicant: Colin Corporation, Komaki-shi, Aichi-ken (JP)
(72) Inventor: Oka, Tohru, Komaki-shi, Aichi-ken (JP); Narimatsu, Kiyoyuki, Komaki-shi, Aichi-ken (JP)
(74) Representative: Winter, Brandl, Fürniss, Hübner, Röss, Kaiser, Polte Partnerschaft

(57) **Abstract**

A method of supplying, from a server which is connected via a communication line to a plurality of member's terminal devices which are respectively operated by a plurality of members who are registered in advance in the server, a set of statistical medical information produced by statistically processing a plurality of sets of medical information obtained from the members and stored in the server, to a user, the method including a medical-information supplying step of selecting, from the plurality of sets of medical information, a set of medical information related to a set of physical information sent to the server from one of the member's terminal devices, operated by one of the members, and supplying the selected set of medical information to the one member, an information storing step of storing the set of physical information of the one member and adding the set of physical information to the set of medical information related to the set of physical information, and a statistically processing step of statistically processing the plurality of sets of medical information and thereby producing the set of statistical medical information.

## Description

### BACKGROUND OF THE INVENTION

### Field of the Invention

The present invention relates to a statistical-medical-information supplying method and a statistical-medical-information supplying apparatus each of which is utilized by members for medical consultation or examination, statistically process the sets of physical information inputted and stored therefor, so as to produce up-to-date statistical medical information, and send the thus produced statistical medical information to users in response to their requests.

### Related Art Statement

When a doctor makes a diagnosis, or makes a decision about treatment, an insurance company calculates a risk, a laboratory determines a theme, or an administration determines a policy about public health, it is very important to obtain statistical medical information. Thus, there have been needs to obtain reliable and up-to-date statistical medical information.

However, since a set of statistical medical information is produced from a number of sets of medical information obtained from a number of living persons, it needs much effort and time to produce the statistical medical information. Thus, many known sets of statistical medical information are out of date or unsatisfactory.

### SUMMARY OF THE INVENTION

It is therefore an object of the present invention to provide a statistical-medical-information supplying method and a statistical-medical-information supplying apparatus that can provide up-to-date and reliable statistical medical information.

The above object has been achieved by the present invention. According to a first aspect of the present invention, there is provided a method of supplying, from a server which is connected via a communication line to a plurality of member's terminal devices which are respectively operated by a plurality of members who are registered in advance in the server, a set of statistical medical information produced by statistically processing a plurality of sets of medical information obtained from the members and stored in the server, to a user, the method comprising: a medical-information supplying step of selecting, from the plurality of sets of medical information, a set of medical information related to a set of physical information sent to the server from one of the member's terminal devices, operated by one of the members, and supplying the selected set of medical information to the one member, an information storing step of storing the set of physical information of the one member and adding the set of physical information to the set of medical information related to the set of physical information, and a statistically processing step of statistically processing the plurality of sets of medical information and thereby producing the set of statistical medical information.

According to this invention, when the members input respective sets of physical information to obtain, by telediagnosis, respective sets of medical information related to the sets of physical information, the sets of physical information are stored at the storing step and are added to the sets of medical information, and the sets of medical information are statistically processed, at the statistically processing step, to obtain statistical medical information. Therefore, it is possible to obtain the up-to-date and reliable statistical medical information. This statistical medical information can be supplied to, and utilized by, a hospital to make a diagnosis or make a decision on medical treatment; by an insurance company to calculate a risk; by a laboratory to determine a theme; or by an administration to make a political decision on public health.

According to a preferred feature of the first aspect of the present invention, the statistical-medical-information supplying method further comprises an outputting step of outputting, when an identification code prepared for the user is sent to the server from a user's terminal device which is operated by the user and which is connected via the communication line to the server, the set of statistical medical information to the user's terminal device.

According to this feature, even if the user may live at a remote place, the user can easily obtain the up-to-date and reliable statistical medical information.

According to another feature of the first aspect of the present invention, the statistical-medical-information supplying method further comprises a charging step of charging the user when the set of statistical medical information is outputted to the user's terminal device.

According to this feature, since the user is charged for the use of the up-to-date and reliable statistical medical information, the present statistical-medical-information supplying method can be carried out on an economical basis. Therefore, the membership fee or the access fee can be zeroed or reduced. This leads to increasing the total number of members, and thereby increasing the degree of reliability of statistical medical information.

According to another feature of the first aspect of the present invention, the statistical-medical-information supplying method further comprises a diagnosing step of making a diagnosis about a physical condition of the one member based on the set of physical information of the one member sent to the server from the one member's terminal device, wherein the medical-information supplying step comprises supplying, to the one member, the selected set of medical information including the made diagnosis.

According to this feature, the medical information supplied to the member includes not only the physical information of the member but also the diagnosis made based on the physical information. Therefore, the member can more accurately recognize his or her physical condition and can obtain the reliable diagnosis. In addition, even if the member may live at a remote place, the member can easily and automatically obtain the diagnosis made about his or her physical condition. Thus, a virtual hospital is established. The virtual hospital can save the time needed for the member living at the remote place to go to an actual hospital.

According to a second aspect of the present invention, there is provided a medical-information supplying apparatus including a server which is connected via a communication line to a plurality of member's terminal devices which are respectively operated by a plurality of members who are registered in advance in the server, and supplying, from the server, a set of statistical medical information produced by statistically processing a plurality of sets of medical information obtained from the members and stored in the server, to a user, the apparatus comprising a medical-information supplying means for selecting, from the plurality of sets of medical information, a set of medical information related to a set of physical information sent to the server from one of the member's terminal devices, operated by one of the members, and supplying the selected set of medical information to the one member, an information storing means for storing the set of physical information of the one member and adding the set of physical information to the set of medical information related to the set of physical information, and a statistically processing means for statistically processing the plurality of sets of medical information and thereby producing the set of statistical medical information.

According to this invention, when the members input respective sets of physical information to obtain, by telediagnosis, respective sets of medical information related to the sets of physical information, the storing means stores the sets of physical information and adds the sets of physical information to the sets of medical information, and the statistically processing means statistically processes the sets of medical information to obtain statistical medical information. Therefore, it is possible to obtain the up-to-date and reliable statistical medical information. This statistical medical information can be supplied to, and utilized by, a hospital to make a diagnosis or make a decision on medical treatment; by an insurance company to calculate a risk; by a laboratory to determine a theme; or by an administration to make a political decision on public health.

According to a preferred feature of the second aspect of the present invention, the statistical-medical-information supplying apparatus further comprises an outputting means for outputting, when an identification code prepared for the user is sent to the server from a user's terminal device which is operated by the user and which is connected via the communication line to the server, the set of statistical medical information to the user's terminal device.

According to this feature, even if the user may live at a remote place, the user can easily obtain the up-to-date and reliable statistical medical information.

According to another feature of the second aspect of the present invention, the statistical-medical-information supplying apparatus further comprises a charging means for charging the user when the set of statistical medical information is outputted to the user's terminal device.

According to this feature, since the user is charged for the use of the up-to-date and reliable statistical medical information, the present statistical-medical-information supplying apparatus can be run on an economical basis. Therefore, the membership fee or the user-related fee can be reduced or even zeroed. This leads to increasing the total number of members, and thereby increasing the degree of reliability of statistical medical information.

According to another feature of the second aspect of the present invention, the statistical-medical-information supplying apparatus further comprises a diagnosing means for making a diagnosis about a physical condition of the one member based on the set of physical information of the one member sent to the server from the one member's terminal device, wherein the medical-information supplying means supplies, to the one member, the selected set of medical information including the made diagnosis.

According to this feature, the medical information supplied to the member includes not only the physical information of the member but also the diagnosis made based on the physical information. Therefore, the member can more accurately recognize his or her physical condition and can obtain the reliable diagnosis. In addition, even if the member may live at a remote place, the member can easily and automatically obtain the diagnosis made about his or her physical condition. Thus, a virtual hospital is established. The virtual hospital can save the time needed for the member living at the remote place to go to an actual hospital.

According to another feature of the second aspect of the present invention, the medical-information supplying apparatus further comprises a plurality of physical-information obtaining devices which are connected to the member's terminal devices, respectively, and which obtain respective sets of physical information from the members and supply the respective sets of physical information to the member's terminal devices.

According to this feature, the members need not input manually the respective sets of physical information into the respective member's terminal devices. In addition, a diagnosis made based on each set of physical information can enjoy a high reliability.

### BRIEF DESCRIPTION OF THE DRAWINGS

The above and optional objects, features, and advantages of the present invention will be better understood by reading the following detailed description of the preferred embodiments of the invention when considered in conjunction with the accompanying drawings, in which:
Fig. 1 is a view for explaining the construction of a statistical-medical-information supplying system as a statistical-medical-information supplying apparatus utilizing a communication line, to which the present invention is applied;
Fig. 2 is a block diagram for explaining essential control functions of a server shown in Fig. 1;
Fig. 3 is a view showing an example of a set of medical information that is supplied from the server of Fig. 1 to a member;
Fig. 4 is a flow chart representing a portion of the essential control functions of the server of Fig. 1, i.e., a virtual-hospital controlling routine; and
Fig. 5 is a flow chart representing another portion of the essential control functions of the server of Fig. 1, i.e., a statistical-medical-information-supply controlling routine.

### DETAILED DESCRIPTION OF PREFERRED EMBODIMENTS

Hereinafter, there will be described a preferred embodiment of the present invention in detail by reference to the drawings.

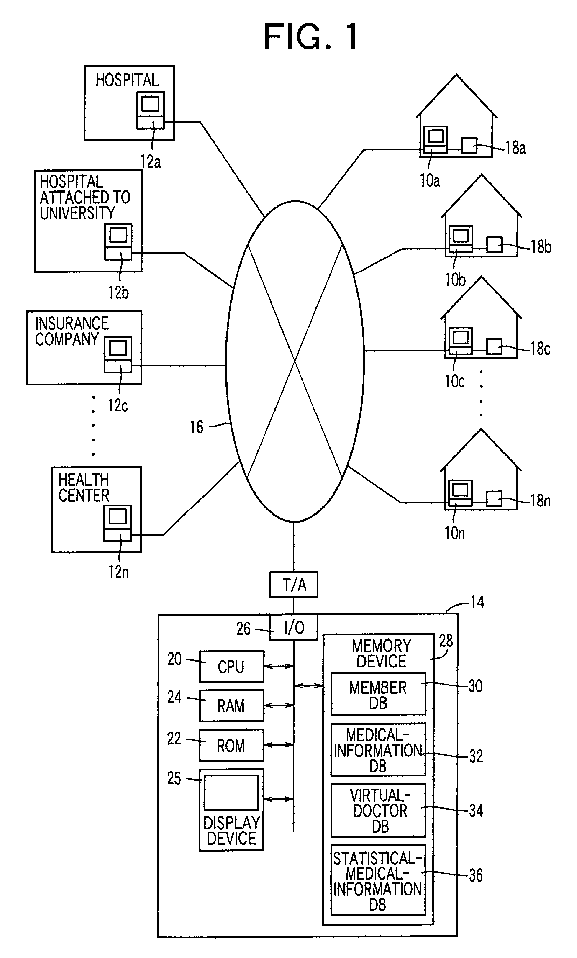

Fig. 1 is a view for explaining the construction of a medical-information supplying system as a medical-information supplying apparatus to which the present invention is applied. As shown in Fig. 1, the present medical-information supplying system includes a plurality of stationary-type or portable-type member's terminal devices 10a, 10b, 10c, ..., 10n that are operable by respective living persons as respective members. The member's terminal devices may be television sets, personal computers, or portable telephones that have the function of making communications via the internet. The supplying system additionally includes a plurality of dealer's or user's terminal devices 12a, 12b, 12c, ..., 12n that are operable by respective dealers or users and are provided in hospitals, university's hospitals, insurance companies, or health centers; and a server 14 as a virtual hospital that is provided in a medical-information supplying company. The supplying system further includes a communication line 16, such as wire or wireless internet (i.e., communication network) or wire or wireless telephone line, that connects the member's terminal devices 10n, the user's terminal devices 12n, and the server 14; with one another, so that highly secret codes, such as SSL, can be communicated among those elements 10n, 12n, 14. The member's terminal devices 10n and the user's terminal devices 12n are provided by, e.g., personal computers each including a display device, and the server 14 is provided by, e.g., a high-speed and high-capacity electronic computer.

A plurality of physical-information obtaining devices 18a, 18b, 18c, ..., 18n are connected to the plurality of member's terminal devices 10a, 10b, 10c, ..., 10n, respectively. Each of the physical-information obtaining devices 18 periodically obtains, from a corresponding one of the members (i.e., living persons), various sorts of physical information including blood pressure BP, weight W, heart rate HR, electrocardiograph waveform ECG, temperature TB, autonomic-nerve activity, pulse-wave propagation velocity PWV, augmentation index AI, eye sight, blood sugar, allergy, and genes. Each of the physical-information obtaining devices 18 may include a plurality of sensors that detect the above-indicated various sorts of physical information, respectively, or an input device that is manually operable by a living person to input the above-indicated various sorts of physical information. The physical-information obtaining devices 18n supply the thus obtained respective sets of physical information to the member's terminal devices 10n, respectively. The blood pressure BP, e.g., systolic and diastolic blood pressure values, may be detected in such a manner that each of the physical-information obtaining devices 18n employs an inflatable cuff, the cuff is wound around an upper arm of the corresponding living person, and blood pressure values of the person are determined, according to so-called oscillometric method, based on change of respective amplitudes of respective heartbeat-synchronous pulses of a cuff pulse wave detected as a pressure signal by a pressure sensor from the cuff during changing of the pressing pressure of the cuff. Alternatively, the blood pressure may be obtained in such a manner that blood pressure values measured using a common sphygmomanometer are inputted through operation of keys of an input device. The weight W may be detected in such a manner that each of the physical-information obtaining devices 18n employs a weight sensor, and a weight of the corresponding living person is detected by the weight sensor, or alternatively the weight W may be obtained in such a manner that a weight measured using a common scales is inputted through operation of keys of an input device. The heart rate HR may be calculated based on the number of the heartbeat-synchronous pulses of the above-described cuff pulse wave that are produced in unit time, or based on the number of heartbeat-synchronous pulses of an electrocardiograph waveform that are produced in unit time and are detected by an electrocardiograph device, not shown. The electrocardiograph waveform ECG may be detected by an electrocardiograph device, not shown. The temperature TB may be detected by a temperature sensor, not shown, that is supported by the above-described cuff and is worn with the cuff on each living person. The autonomic-nerve activity may be determined based on fluctuations of the heart rate values HR or the blood pressure values BP. The pulse-wave propagation velocity PWV may be determined based on a propagation time from the time when a second heart sound II is detected by a heart-sound microphone to the time when a dicrotic notch of a carotid pulse wave is detected, and a propagation distance between the heart and the carotid artery. The augmentation index AI may be defined as a ratio or proportion of one of an amplitude of an incident-wave component of an arterial pulse wave, such as carotid pulse wave, and an amplitude of a reflected-wave component of the pulse wave, to the other, and may be determined based on the waveform of the carotid pulse wave or the cuff pulse wave.

The server 14 is essentially provided by a so-called microcomputer including a CPU (central processing unit) 20, a ROM (read only memory) 22 that stores control programs, a RAM (random access memory) 24 that functions as a temporary-storage device, a display device 25, and an input-and-output interface 26 that is connected via a terminal adaptor TA to the communication line 16. The server 14 includes a memory device 28 that stores various sorts of data bases (DB) corresponding to various sorts of information. More specifically described, the memory device 28 includes a member DB (data base) 30 that stores respective names of a plurality of living persons who have contracted, at their own charge or no charge, with the medical-information supplying company to become, in advance, members who are to be supplied with medical information from the company; respective identification codes (ID) of the members; respective pass codes or words of the members; and respective names and identification codes (ID) of the dealers or users. The memory device 28 additionally includes a medical-information DB (data base) 32 that stores sets of medical information including the respective sets of physical information sent from the members, and respective medical evaluations or diagnoses automatically made based on those sets of physical information. The memory device 28 further includes a virtual-doctor DB (data base) 34 that stores a diagnosing program for use in automatically making those medical evaluations or diagnoses on the sets of physical information or respective changes (or trends) of the sets of information; and a statistical-medical-information DB (data base) 36 that stores statistical medical information obtained by statistically processing the sets of medical information, stored in the medical-information DB 32, that includes the respective sets of physical information sent from the members, and the respective medical evaluations or diagnoses automatically made on those sets of physical information.

Thus, the member's terminal devices 10n are operated by the respective members who have been registered in the server 14, and each one of the member's terminal devices 10n periodically sends a set of physical information of a corresponding one of the members to the server 14. At that time, the server 14 stores the set of physical information, and automatically makes, according to the diagnosing program pre-stored in the virtual-doctor DB 34, a medical evaluation or diagnosis on the set of physical information or the change or trend of the set of physical information from the past sets of physical information stored in the medical-information DB 32. In addition, the server 14 sends the thus made medical evaluation or diagnosis, together with the current and past sets of physical information of the member, to the member's terminal device 10n, so that the medical evaluation or diagnosis and the sets of physical information are displayed on the display device of the terminal device 10n of the member. Meanwhile, the user's terminal devices 10n may be operated by the respective users using the respective identification codes ID that have been received from the medical-information supplying company when the users contracted with the company and have been registered in the server 14, for example, for the purpose of obtaining the sets of statistical medical information stored in the statistical-medical-information DB 36. At that time, the server 14 reads, from the statistical-medical-information DB 36, one or more sets of statistical medical information requested by the user, and sends the thus obtained set or sets of statistical medical information to the user's terminal device 12n, so that the set or sets of statistical medical information is or are displayed on the display device of the terminal device 12n.

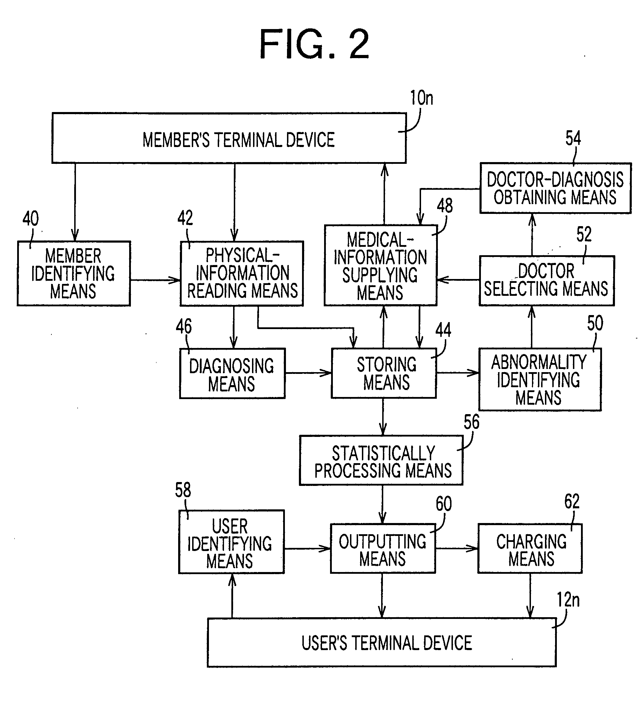

Fig. 2 is a block diagram for explaining the essential control functions of the above-described server 14. A member identifying means 40 judges whether an identification code ID inputted and sent by an arbitrary one of the member's terminal devices 10n is identical with one of the identification codes ID pre-stored in the member DB 30, and thereby judges whether a living-person who has made access to the server 14 is one of the members pre-registered in the server 14. A physical-information reading means 42 reads, when the member identifying means 40 has identified the person as one of the registered members, the set of physical information sent by the member's terminal device 10n, and a storing means 44 stores, in the medical-information DB 32, the thus read set of physical information in such a manner that the set of physical information read is associated with the identification code ID of the member identified. A diagnosing means 46 automatically makes a diagnosis on the set of physical information read by the reading means 42, according to the diagnosing program pre-stored in the virtual-doctor DB 34. For example, the diagnosing means 46 judges whether the set of physical information read by the reading means 42 falls in a predetermined reference range, and thereby makes a medical evaluation or diagnosis. The storing means 44 stores, in the medical-information DB 32, the thus made medical evaluation or diagnosis such that the medical evaluation or diagnosis is associated with the identification code ID or the set of physical information. A medical-information supplying means 48 supplies, to the member's terminal device 10n that has sent the current set of physical information, the current and past sets of physical information, and the medical evaluation or diagnosis made on those sets of physical information, all of which have been stored by the storing means 44 in the medical-information DB 32, so that the trend of physical information and the medical evaluation or diagnosis are displayed on the member's terminal device 10n.

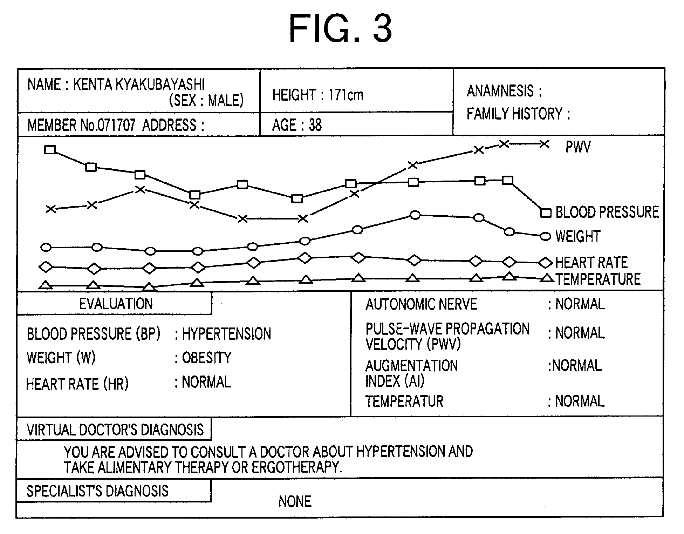

An abnormality identifying means 50 judges whether each set of physical information read by the reading means 42, or an amount of change of the set of physical information, falls outside a predetermined normal range and thereby judges whether each member is abnormal. The normal range may be identical with the above-described reference range. A doctor selecting means 52 selects, when the abnormality identifying means 50 judges based on the set of physical information that the member is abnormal, an appropriate one of the doctors who are listed and stored in the memory device 28, based on the sort of the physical information judged as abnormal, and an address of the abnormal member, such that the selected doctor is specialized in the sort of the abnormality and is near to the address of the abnormal member. A doctor-diagnosis obtaining means 54 sends the physical information judged as abnormal, to the doctor selected by the doctor selecting means 52, requests the doctor to make a diagnosis about the set of physical information, and receives the diagnosis made by the doctor. The storing means 44 stores, in the medical-information DB 32, the diagnosis made by the doctor, such that the diagnosis is associated with the identification code ID or the physical information, and the medical-information supplying means 48 sends the diagnosis to the member. Fig. 3 shows an example of a set of medical information that is displayed on the display device of the member's terminal device 10n that has sent the set of physical information to the server 14. In this way, each member can send his or her own physical information via the member's terminal device 10n, so as to obtain the automatically made diagnosis and additionally obtain, when the physical information is abnormal, the diagnosis made by the actual doctor on the physical information. Thus, the member can go to the "virtual" hospital to undergo the medical examination, and even if the member may live at a remote place from the virtual hospital or the server 14, the member can obtain the results as if he or she had undergone an actual examination at an actual hospital.

A statistically processing means 56 statistically processes, under one or more given conditions, each of the various sorts of physical information that have been inputted by the members via the respective member's terminal devices 10n into the above-described virtual-hospital system, that is, the sets of physical information and the automatic evaluations or diagnoses that have been stored in the medical-information DB 32, produces various sets of statistical medical information, and stores those sets of statistical medical information in the statistical-medical-information DB 36. For example, the statistically processing means 56 calculates, based on the data stored in medical-information the DB 32, an average blood pressure or an average eye sight for each age, each sex, each area, each weight, or each year, calculates respective incidence rates of hypertension, arteriosclerosis, arteriostenosis, diabetes, asthma, and atopy, and determines, for each age, each sex, each area, each weight, or each year, the examination item that is most frequently performed in connection with each symptom. Those data are stored in the statistical-medical-information DB 36.

A user identifying means 58 judges whether a user's name and a user's identification code ID inputted and sent by one of the user's terminal devices 12n are identical with respective ones of the user's names and the user's identification codes ID pre-stored (i.e., pre-registered) in the member DB 30, and thereby judges whether a living person who has made access to the server 14 is one of the users registered in the server 14. An outputting means 60 judges, when the user identifying means 58 has identified the person as one of the registered users, that the user is requesting statistic medical information, searches, in the statistical-medical-information DB 36, one or more sorts of statistical medical information requested by the user, and outputs (i.e., sends) the searched statistical medical information to the user's terminal device 12n of the user. For example, if the user requests statistical medical information about incidence rate of diabetes of 25-year-old men, the outputting means 60 sends an average incidence rate of diabetes of 25-year-old men in all areas; if the user requests information about an average blood pressure of 20-year-old women in Tokyo and Nagano Prefectures, the outputting means 60 outputs the average blood pressure of 20-year-old women in Tokyo and Nagano Prefectures; and if the user requests information about an examination item performed for four-year-old boys who suffer serious cough, the output means 60 sends the examination item most frequently performed for four-year-old boys. In each case, the statistical medical information is displayed on the display device of the user's terminal device 12n. A charging means 62 charges the user or dealer who operates the user's terminal device 12n that has made access to the server 14, for the use of the statistical medical information. The amount of charging depends on the sort of the statistic medical information requested and the time or times of access to the server 14. The charging means 62 produces data to issue a bill and sends the data to the user's terminal device 12n so that the data are displayed or outputted by the terminal device 12n.

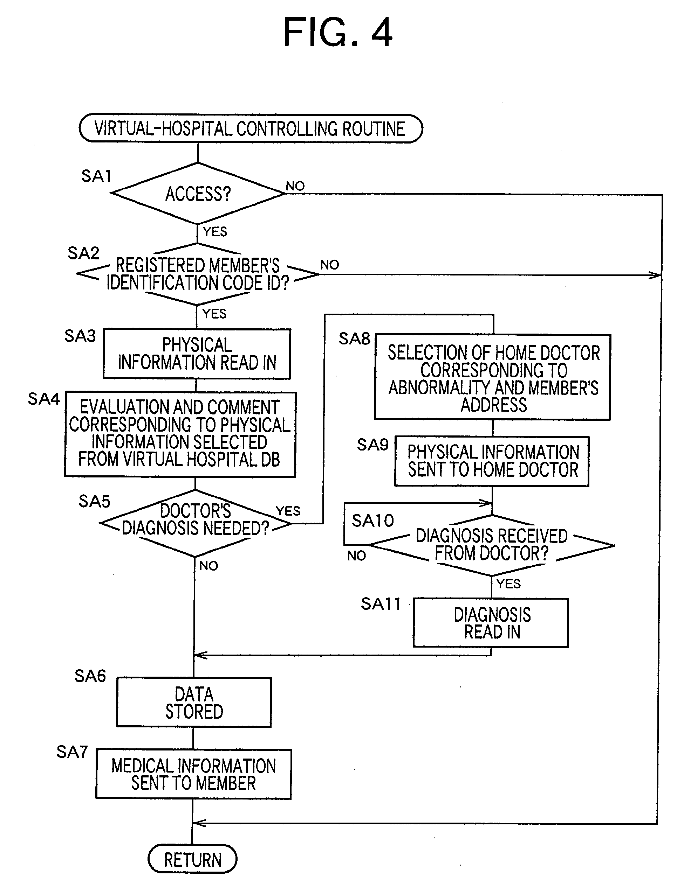

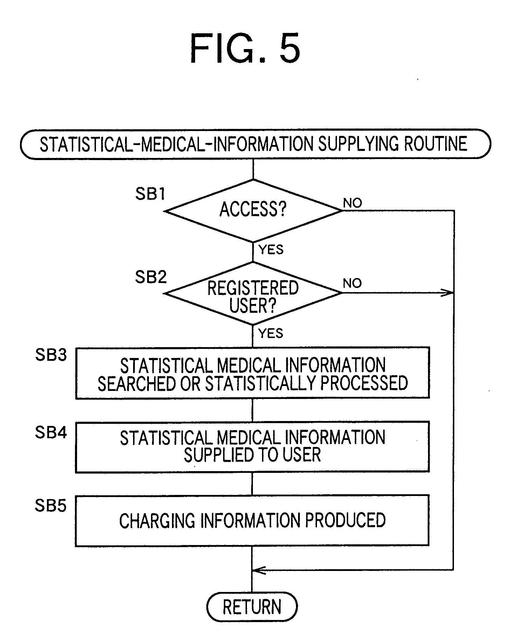

Figs. 4 and 5 show respective flow charts representing the essential control functions of the above-described server 14, i.e., Fig. 4 is a virtual-hospital controlling routine and Fig. 5 is a statistical-medical-information supplying routine. These routines are iteratively executed at a short period.

At Step SA1 of Fig. 4 (hereinafter, "Step(s)" is omitted), the server 14 judges whether any one of the member's terminal devices 10n has made access to the server 14. If a negative judgment is made at SA1, this routine is quitted. On the other hand, if a positive judgment is made at SA1, the control goes to SA2 corresponding to the member identifying step or the member identifying means 40. At SA2, the server judges whether the member's identification code ID inputted by the member's terminal device 10n is identical with one of the member's identification codes ID registered in advance in the server. If a negative judgment is made at SA2, this routine is quitted. On the other hand, if a positive judgment is made at SA 2, the control goes to SA3 corresponding to the physical-information reading step or the physical-information reading means 42. At SA3, the server reads at least one sort of physical information that has been obtained by the physical-information obtaining device 18n connected to the member's terminal device 10n and has been sent from the terminal device 10n, i.e., at least one of blood pressure BP, weight W, heart rate HR, electrocardiograph waveform ECG, temperature TB, autonomic-nerve activity, pulse-wave propagation velocity PWV, augmentation index AI, eye sight, blood sugar, allergy, and genes.

Then, the control goes to SA4 corresponding to the diagnosing step or the diagnosing means 46. At SA4, the server automatically makes, according to the automatically evaluating and diagnosing program stored in the virtual-doctor DB 34, a diagnosis about the new set of physical information, the past set or sets of physical information, and the change of the new and past sets of physical information. For example, the server selects one of a plurality of evaluations and/or comments that corresponds to the new set of physical information. Fig. 3 shows the thus made evaluations and the virtual doctor's diagnosis. Then, the control goes to SA5 corresponding to the abnormality identifying step or the abnormality identifying means 50. At SA5, the server judges whether it is needed to consult an actual doctor or a specialist. More specifically described, the server judges whether the new set of physical information falls outside a predetermined normal range. If a negative judgment is made at SA5, the control goes to SA6 corresponding to the storing step or the storing means 44. At SA6, the server stores, in the medical-information DB 32, the new set of physical information and the automatically made evaluation and/or diagnosis, such that those medical information is associated with the identification code ID of the member. Then, the control goes to SA7 corresponding to the medical-information supplying step or the medical-information supplying means 48. At SA7, the server sends, to the member's terminal device 10n that has sent the new set of physical information, a set of medical information that has been stored, for the member, in the medical-information DB 32, that is, the new and past sets of physical information (i.e., the trend of physical information) and the automatic evaluation and/or diagnosis, so that the set of medical information is displayed on the display device of the member's terminal device 10n, as shown in Fig. 3. Thus, each member can easily and economically request the virtual hospital or doctor to check his or her physical condition or make a diagnosis on it.

On the other hand, if a positive judgment is made at SA5, the control goes to SA8 corresponding to the doctor selecting step or the doctor selecting means 52. At SA8, the server selects, from a pre-stored list of doctors, a doctor or a specialist who is appropriate for the sort of the abnormal physical information, the degree of abnormality of the physical information, and the address of the member. For example, a specialist who is specialized in the sort of the abnormal physical information, or a doctor belonging to a medical institution located in an area where the member can go. Next, the control goes to SA 9 to SA11 corresponding to the doctor-diagnosis obtaining step or the doctor-diagnosis obtaining means 54. First, at SA9, the server sends, to the selected doctor, the abnormal, new set of physical information of the member and the past sets of physical information of the same, and requests the doctor to make a diagnosis on the sets of physical information. Then, at SA10, the server judges whether the server has received a diagnosis sent from the doctor. SA10 is repeated till a positive judgment is made. When a positive judgment is made at SA10, the control goes to SA11 to read in the diagnosis sent from the doctor. Subsequently, the control goes to SA6 and SA7. At SA6, the server stores, in the medical-information DB 32, not only the new set of physical information and the automatic evaluation or diagnosis, but also the diagnosis made by the actual doctor, such that those medical information is associated with the identification code ID of the member. Then, the control goes to SA7 corresponding to the medical-information supplying step or the medical-information supplying means 48. At SA7, the server sends, to the member's terminal device 10n that has sent the new set of physical information, a set of medical information that has been stored, for the member, in the medical-information DB 32, that is, the new and past sets of physical information (i.e., the trend of physical information), the automatic evaluation or diagnosis, and the diagnosis made by the actual doctor, so that the set of medical information is displayed on the member's terminal device 10n, as shown in Fig. 3, and additionally the diagnosis made by the actual doctor, and the name and address of the hospital to which the doctor belongs, or the address of the doctor are indicated in an area prepared therefor (i.e., the lowermost area). Thus, each member can easily and economically request the virtual hospital or doctor to check his or her physical condition or make a diagnosis on it, and additionally can obtain a diagnosis made by the actual doctor when the new set of physical information is judged as abnormal. If necessary, the member can request the doctor to re-examine his or her physical condition.

In Fig. 5, at SB1, the server judges whether any one of the respective user's terminal devices 12n has made access to the server. If a negative judgment is made at SB1, this routine is quitted. On the other hand, if a positive judgment is made at SB1, the control goes to SB2 corresponding to the user identifying step or the user identifying means 58. At SB2, the server judges whether the user's identification code ID inputted by the user's terminal device 12n is identical with one of the user's identification codes ID pre-registered in the server. If a negative judgment is made at SB2, this routine is quitted. On the other hand, if a positive judgment is made at SB2, the control goes to SB3 corresponding to the statistically processing means 56. At SB3, the server searches, in the statistical-medical-information DB 36, one or more sorts of statistical medical information requested by the user, or newly processes the data stored in the medical-information DB 32 to produce one or more sorts of statistical medical information requested by the user. Then, the control goes to SB4 corresponding to the outputting step or the outputting means 60. At SB4, the statistic medical information obtained at SB3 is sent to the user's terminal device 12n that has made access to the server 14. Next, the control goes to SB5 corresponding to the charging step or the charging means 62. At SB5, the server produces charging information to charge the user's terminal device 12n, that is, the user who has made access to the server to obtain the statistic medical information. At a step, not shown, the server automatically issues, based on the charging information, a bill to charge the above-indicated user. The amount of charging and the address to which the bill is sent may be determined in a contract made between the server and the user, or may be noticed to, or inputted by, the user on the display of the user's terminal device 12n.

As is apparent from the foregoing description of the illustrated embodiment, the individual living persons as the individual members input their physical information to obtain, by telediagnosis, their medical information produced in relation with their physical information, the storing means 44 stores the thus produced medical information in the medical-information DB 32, and the statistically processing means 56 (SB3) statistically processes the thus stored medical information to obtain statistical medical information. Therefore, when a user, such as a hospital, a university (or, a hospital attached thereto), an insurance company, a laboratory attached to a company, or a health center, sends its identification code ID to the server 14, the user can receive, from the server 14, the up-to-date and reliable statistical medical information. This statistical medical information can be utilized by a hospital to make a diagnosis or make a decision on medical treatment, by an insurance company to calculate a risk; by a laboratory to make a decision on theme; or by an administration to make a political decision on public health.

In addition, in the illustrated embodiment, when the identification code ID pre-registered for each of the users is inputted and is identified, the outputting means 60 (SB5) sends the statistical medical information produced by the statistically processing means 56 (SB3), to the terminal device 12n of the user. Thus, even if the user may live at a remote place, the user can easily obtain the up-to-date and reliable statistical medical information.

In the illustrated embodiment, when the outputting means 58 outputs, at the outputting step SB4, the statistical medical information produced by the statistically processing means 56 (SB3), the charging means 62 charges, at the charging step SB5, the user who operates the user's terminal device 12n to which the statistical medical information is sent. That is, since the user is charged for the use of the up-to-date and reliable statistical medical information, the company can run the present statistical-medical-information supplying system on an economical basis. Therefore, the membership fee or the access fee can be zeroed or reduced. This leads to increasing the total number of members, and thereby increasing the degree of reliability of statistical medical information.

In addition, in the illustrated embodiment, the diagnosing means 46 (SA4) makes a diagnosis about a physical condition of each individual member based on a new set of physical information inputted through the member's terminal devices 10n operated by the each member, and the medical-information supplying means 48 (SA7) supplies, to the each member, a set of medical information including the diagnosis made by the diagnosing means 46. Thus, the medical information supplied to the individual member includes not only the physical information of the member but also the diagnosis made based on physical information. Therefore, the individual member can more accurately recognize his or her physical condition and can obtain the reliable diagnosis. In addition, even if the member may live at a remote place, the member can easily and automatically obtain the diagnosis made about his or her physical condition. Thus, a virtual hospital is established. The virtual hospital can save the time needed for the member living at the remote place to go to an actual hospital.

In the illustrated embodiment, the physical-information obtaining devices 18n are connected to the member's terminal devices 10n, respectively, and obtain respective sets of physical information from the members and supply the respective sets of physical information to the member's terminal devices. Thus, the members need not input manually the respective sets of physical information into the respective member's terminal devices 10n. In addition, a diagnosis made based on each set of physical information enjoys a high reliability.

While the present invention has been described in its embodiment by reference to the drawings, it is to be understood that the present invention can otherwise be embodied.

For example, in the illustrated embodiment, the server 14 is described such that the server 14 is provided by a single computer. However, the server 14 may be provided by a plurality of computers, and those computers may be provided at respective positions remote from each other.

In the illustrated embodiment, each of the physical-information obtaining devices 18n connected to the member's terminal devices 10n has the functions of obtaining blood pressure BP, weight W, heart rate HR, electrocardiograph waveform ECG, temperature TB, autonomic-nerve activity, pulse-wave propagation velocity PWV, augmentation index AI, etc. However, each physical-information obtaining device 18n may be one that can obtain only a portion (one, two, ..., but not all) of the above-indicated sorts of physical information, or one that can obtain other sorts of physical information. The sorts of physical information that are not directly detected by each physical-information obtaining device 18n may be manually inputted through each member's terminal device 10n after having been measured using scales, a sphygmomanometer, a heart-rate meter, an electrocardiograph, etc. In this sense, each physical-information obtaining device 18n may be one that does not include any sensors.

In the flow charts shown in Figs. 4 and 5, the order of the steps may be changed, as needed. In addition, SA5 to SA11 of Fig. 4 may be omitted.

In the illustrated embodiment, the statistically processing means 56 may be one that produces one or more sorts of statistical medical information requested by the user, on a real-time basis, that is, in response to each access of the user, or one that produces, in advance, various sorts of statistical medical information, and stores the thus produced sorts of statistical medical information in a memory. In the latter case, in response to each access of the user, the outputting means 60 searches, in the memory, one or more sorts of statistical medical information requested by the user, and outputs the searched statistical medical information to the user.

While the present invention has been described in detail in its embodiments by reference to the drawings, it is to be understood that the present invention is not limited to the details of the described embodiments but may be embodied with various changes or improvements that may occur to a person skilled in the art.

## Claims

1. A method of supplying, from a server which is connected via a communication line to a plurality of member's terminal devices which are respectively operated by a plurality of members who are registered in advance in the server, a set of statistical medical information produced by statistically processing a plurality of sets of medical information obtained from the members and stored in the server, to a user, the method comprising:
a medical-information supplying step of selecting, from the plurality of sets of medical information, a set of medical information related to a set of physical information sent to the server from one of the member's terminal devices, operated by one of the members, and supplying the selected set of medical information to said one member,
an information storing step of storing the set of physical information of said one member and adding the set of physical information to the set of medical information related to the set of physical information, and
a statistically processing step of statistically processing the plurality of sets of medical information and thereby producing the set of statistical medical information.

2. A method according to claim 1, further comprising an outputting step of outputting, when an identification code prepared for said user is sent to the server from a user's terminal device which is operated by said user and which is connected via the communication line to the server, the set of statistical medical information to the user's terminal device.

3. A method according to claim 2, further comprising a charging step of charging said user when the set of statistical medical information is outputted to the user's terminal device.

4. A method according to claim 1, further comprising a diagnosing step of making a diagnosis about a physical condition of said one member based on the set of physical information of said one member sent to the server from said one member's terminal device, wherein the medical-information supplying step comprises supplying, to said one member, the selected set of medical information including the made diagnosis.

5. A medical-information supplying apparatus including a server (14) which is connected via a communication line (16) to a plurality of member's terminal devices (10) which are respectively operated by a plurality of members who are registered in advance in the server, and supplying, from the server, a set of statistical medical information produced by statistically processing a plurality of sets of medical information obtained from the members and stored in the server, to a user, the apparatus comprising:
a medical-information supplying means (48) for selecting, from the plurality of sets of medical information, a set of medical information related to a set of physical information sent to the server (14) from one (10n) of the member's terminal devices (10), operated by one of the members, and supplying the selected set of medical information to said one member,
an information storing means (40, 42, 44) for storing the set of physical information of said one member and adding the set of physical information to the set of medical information related to the set of physical information, and
a statistically processing means (56) for statistically processing the plurality of sets of medical information and thereby producing the set of statistical medical information.

6. An apparatus according to claim 5, further comprising an outputting means (58, 60) for outputting, when an identification code prepared for said user is sent to the server (14) from a user's terminal device (12n) which is operated by said user and which is connected via the communication line (16) to the server, the set of statistical medical information to the user's terminal device.

7. An apparatus according to claim 6, further comprising a charging means (62) for charging said user when the set of statistical medical information is outputted to the user's terminal device.

8. An apparatus according to claim 5, further comprising a diagnosing means (46) for making a diagnosis about a physical condition of said one member based on the set of physical information of said one member sent to the server (14) from said one member's terminal device (10n), wherein the medical-information supplying means (48) supplies, to said one member, the selected set of medical information including the made diagnosis.

9. An apparatus according to claim 5, further comprising a plurality of physical-information obtaining devices (18) which are connected to the member's terminal devices (10), respectively, and which obtain respective sets of physical information from the members and supply the respective sets of physical information to the member's terminal devices.
